# EUROPÄISCHE PATENTSCHRIFT

(11) **EP 4 374 815 B1**
(45) Veröffentlichungstag und Bekanntmachung des Hinweises auf die Patenterteilung: **23.07.2025**
(21) Anmeldenummer: 22209976.4
(22) Anmeldetag: 28.11.2022
(51) Int. Cl.: A61C 5/73, A61C 5/77, A61C 8/00, A61C 13/00, A61C 13/083, A61C 13/09, B33Y 10/00, B33Y 30/00, B33Y 70/00

(54) **VERFAHREN ZUM HERSTELLEN EINER DENTALEN RESTAURATION**
METHOD FOR PRODUCING A DENTAL RESTORATION
PROCÉDÉ DE FABRICATION D'UNE RESTAURATION DENTAIRE

(43) Veröffentlichungstag der Anmeldung: 29.05.2024
(73) Patentinhaber: Ivoclar Vivadent AG, 9494 Schaan (LI)
(72) Erfinder: Atilim, Eser, 88131 Lindau (DE); Hendrik, John, 9470 Buchs (CH)
(74) Vertreter: Baldus, Oliver

(56) Entgegenhaltungen:
- US-A1- 2007 015 110
- US-A1- 2015 320 525

## Beschreibung

Die vorliegende Erfindung betrifft ein Verfahren zum Herstellen einer dentalen Restauration und eine Herstellungsvorrichtung zum Herstellen einer dentalen Restauration.

Bei der digitalen Fertigung von Kronen und Brücken werden pauschale Lastfälle für diese Kronen und Brücken angenommen. Dadurch wird entweder die Anzahl der Brückenglieder eingeschränkt oder diese Teile sind hinsichtlich der Wandstärken überdimensioniert. Dies führt dazu, dass die mechanischen Eigenschaften der verwendeten Materialien um ein Vielfaches über den real auftretenden Belastungen liegen.

Die Druckschrift US 2015/320525 A1 betrifft Verfahren zur Herstellung von Zahnprothesen, bei denen ein Pulver eines Dentalmaterials selektiv geschmolzen wird, um eine dreidimensionale Zahnprothese mit einer gewünschten physikalischen Geometrie und einer oder mehreren funktionell abgestuften mechanischen Eigenschaften herzustellen.

Die Druckschrift US 2007/015110 A1 betrifft Verfahren und Materialien, die zur Herstellung von Zahnprothesen verwendet werden. Ein Insert-Molding-Verfahren kann verwendet werden, um einen Metall- oder Faserverstärkungseinsatz oder -element in einer Prothesenkomponente zu platzieren.

Es ist die technische Aufgabe der vorliegenden Erfindung, die Fertigung von dentalen Restaurationen zu verbessern.

Diese technische Aufgabe wird durch Gegenstände nach den unabhängigen Ansprüchen gelöst. Technisch vorteilhafte Ausführungsformen sind Gegenstand der abhängigen Ansprüche, der Beschreibung und der Zeichnungen.

Gemäß einem ersten Aspekt der Erfindung wird die technische Aufgabe durch ein Verfahren zum Herstellen einer dentalen Restauration gelöst, mit den Schritten eines Bestimmens eines ersten räumlichen Bereiches der dentalen Restauration, der einer höheren Belastung ausgesetzt ist als ein zweiter räumlicher Bereich der dentalen Restauration; und eines Herstellens der dentalen Restauration in dem ersten räumlichen Bereich mit einem anderen Herstellungsmaterial als in dem zweiten räumlichen Bereich, wobei das Herstellungsmaterial in dem ersten räumlichen Bereich anders als in dem zweiten räumlichen Bereich dotiert wird.

Dadurch wird beispielsweise der technische Vorteil erreicht, dass ein Sinterprozess ohne Sinterverzug durchgeführt werden kann und das Herstellungsmaterial unterschiedliche Festigkeitswerte aufweist. Neben den Sintereigenschaften können auch die mechanischen Eigenschaften, die optischen Eigenschaften und die Alterungsbeständigkeit mittels der Dotierung eingestellt werden. Durch das Verfahren können Materialeigenschaften optimal ausgenutzt werden und ein Indikationsbereich erweitert werden. Spannungsspitzen innerhalb der dentalen Restauration können durch eine entsprechende Materialanpassung aufgefangen werden. Die dentale Restauration lässt sich daher mit einem geringeren Materialeinsatz herstellen.

In einer technisch vorteilhaften Ausführungsform des Verfahrens wird der erste räumliche Bereich dadurch bestimmt, dass in diesem räumlichen Bereich die interne Spannung über einem vorgegebenen Wert liegt. Dadurch wird beispielsweise der technische Vorteil erreicht, dass sich der erste räumliche Bereich auf einfache Weise festlegen lässt.

In einer weiteren technisch vorteilhaften Ausführungsform des Verfahrens wird die Belastung mittels einer Finite-Elemente-Methode berechnet. Dadurch wird beispielsweise der technische Vorteil erreicht, dass die Belastung innerhalb der dentalen Restauration mit einer hohen Genauigkeit berechnet werden kann.

In einer weiteren technisch vorteilhaften Ausführungsform des Verfahrens werden vorgegebene Kräfte auf die dentale Restauration für die Finite-Elemente-Methode angewendet. Beispielsweise können die Berechnungen auf Basis von Worst-Case-Scenarios berechnet werden, bei denen maximal zu erwartende Kräfte an den ungünstigsten Punkten der dentalen Restauration angreifen. Dadurch wird beispielsweise der technische Vorteil erreicht, dass die dentale Restauration mit einer hohen Festigkeit (Biaxialfestigkeit, Bruchzähigkeit) berechnet werden kann.

In einer weiteren technisch vorteilhaften Ausführungsform des Verfahrens wird das Herstellungsmaterial für den ersten und/oder zweiten räumlichen Bereich auf Basis einer berechneten Belastung ausgewählt. Dadurch wird beispielsweise der technische Vorteil erreicht, dass für die räumlichen Bereiche unterschiedliche Festigkeiten erreicht und an die Belastung angepasst werden.

Als geeignete Festigkeitswerte (Biegefestigkeit + Bruchzähigkeit) für unterschiedlich yttriumdotierte Zirkondioxid-Materialien sind folgende Werte üblich:

| Material | 3Y-TZP | 4Y-TZP | 5Y-TZP |
|---|---|---|---|
| Biaxiale Biegefestigkeit [MPa] | 1000 ± 200(*) | 750 ± 100(*) | 600 ± 50(*) |
| Bruchzähigkeit [MPa√m] | 5.00 ± 0.25 | 3.75 ± 0.25 | 2.40 ± 0.25 |

In einer weiteren technisch vorteilhaften Ausführungsform des Verfahrens wird in dem ersten räumlichen Bereich ein Herstellungsmaterial mit einer höheren Festigkeit als in dem zweiten räumlichen Bereich verwendet. Dadurch wird beispielsweise der technische Vorteil erreicht, dass eine Wanddicke in diesen Bereichen verringert werden kann.

In einer weiteren technisch vorteilhaften Ausführungsform des Verfahrens wird das Herstellen der dentalen Restauration mittels eines dreidimensionalen Druckverfahrens durchgeführt. Dadurch wird beispielsweise der technische Vorteil erreicht, dass die Herstellung der dentalen Restauration effizient durchgeführt werden kann.

In einer weiteren technisch vorteilhaften Ausführungsform des Verfahrens verwendet das dreidimensionale Druckverfahren einen Freistrahl-Materialauftrag. Dadurch wird beispielsweise der technische Vorteil erreicht, dass sich die Herstellungsmaterialien auf einfache Weise in den jeweiligen räumlichen Bereichen anordnen lassen.

Gemäß einem zweiten Aspekt der Erfindung wird die technische Aufgabe durch eine Herstellungsvorrichtung zum Herstellen einer dentalen Restauration gelöst, mit einer Bestimmungsvorrichtung zum Bestimmen eines ersten räumlichen Bereiches der dentalen Restauration, der einer höheren Belastung ausgesetzt ist als ein zweiter räumlicher Bereich der dentalen Restauration; und einem Herstellungsgerät zum Herstellen der dentalen Restauration in dem ersten räumlichen Bereich mit einem anderen Herstellungsmaterial als in dem zweiten räumlicher Bereich, wobei das Herstellungsgerät ausgebildet ist, das Herstellungsmaterial in dem ersten räumlichen Bereich anders als in dem zweiten räumlichen Bereich zu dotieren. Dadurch wird beispielsweise ebenfalls der technische Vorteil erreicht, dass ein Sinterprozess ohne Sinterverzug durchgeführt werden kann. Durch die Herstellungsvorrichtung werden die gleichen technischen Vorteile wie durch das Verfahren nach dem ersten Aspekt erreicht.

In einer technisch vorteilhaften Ausführungsform der Herstellungsvorrichtung ist die Bestimmungsvorrichtung ausgebildet ist, den ersten räumlichen Bereich dadurch zu bestimmen, dass in diesem räumlichen Bereich die interne Spannung über einem vorgegebenen Wert liegt. Dadurch wird beispielsweise ebenfalls der technische Vorteil erreicht, dass der erste räumliche Bereich auf einfache Weise festgelegt werden kann.

In einer weiteren technisch vorteilhaften Ausführungsform der Herstellungsvorrichtung ist die Bestimmungsvorrichtung ausgebildet, die Belastung mittels einer Finite-Elemente-Methode zu berechnen. Dadurch wird beispielsweise ebenfalls der technische Vorteil erreicht, dass sich die Belastung mit einer hohen Genauigkeit berechnen lässt.

In einer weiteren technisch vorteilhaften Ausführungsform der Herstellungsvorrichtung ist das Herstellungsgerät ausgebildet, das Herstellungsmaterial für den ersten und/oder zweiten räumlichen Bereich auf Basis einer berechneten Belastung auszuwählen. Dadurch wird beispielsweise ebenfalls der technische Vorteil erreicht, dass die Herstellung der dentalen Restauration vereinfacht wird.

In einer technisch vorteilhaften Ausführungsform der Herstellungsvorrichtung umfasst das Herstellungsgerät einen 3D-Drucker. Dadurch wird beispielsweise ebenfalls der technische Vorteil erreicht, dass die Herstellung der dentalen Restauration effizient durchgeführt werden kann.

Ausführungsbeispiele der Erfindung sind in den Zeichnungen dargestellt und werden im Folgenden näher beschrieben.

Es zeigen:
- Fig. 1: eine schematische Ansicht zur Herstellung einer dentalen Restauration;
- Fig. 2: eine schematische Ansicht einer Herstellungsvorrichtung zum Herstellen einer dentalen Restauration; und
- Fig. 3: ein Blockdiagramm eines Verfahrens zum Herstellen der dentalen Restauration.

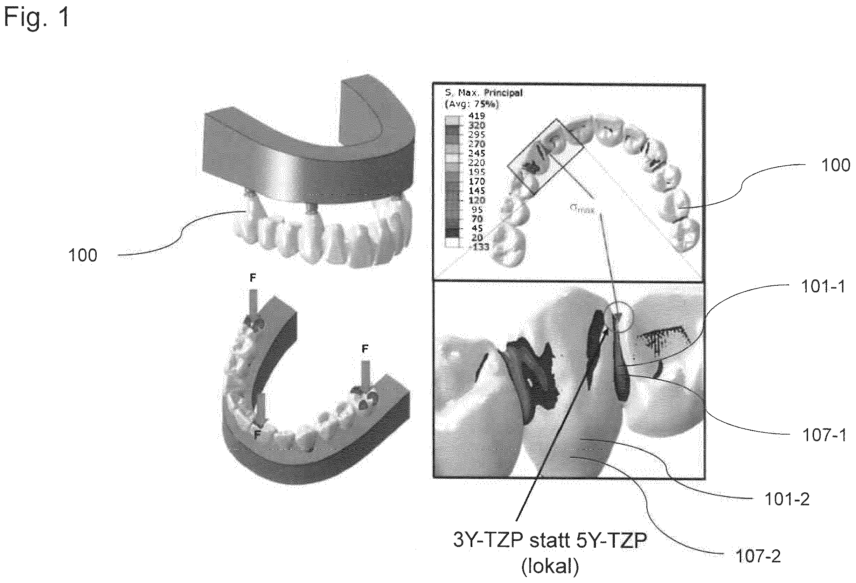

Fig. 1 zeigt eine schematische Ansicht zur Herstellung einer dentalen Restauration 100. Die dentale Restauration 100 ist beispielsweise eine Krone, eine Brücke, eine Teil- oder Vollprothese.

Um die mechanischen Eigenschaften der Herstellungsmaterialien 107-1 und 107-2 besser auszunutzen, werden zunächst über eine FEM-Software (Finite-Elemente-Methode-Software) vorgegebene Lastfälle für die dentale Restauration 100 simuliert. Durch die FEM-Software können interne Kraftverläufe und Spannungen auf Basis eines dreidimensionalen Modells der dentalen Restauration 100 berechnet werden, um Schwachstellen in der dentalen Restauration 100 zu bestimmen. Zur einfacheren Identifizierung der unter- oder überbelasteten räumlichen Bereiche (Subvolumen) können diese über eine spezifische Einfärbung gekennzeichnet werden. Beispielsweise lassen sich auf diese Weise innerhalb der dentalen Restauration 100 räumliche Bereiche 101-1 bestimmen, in denen eine interne Spannung über einem vorgegebenen Wert liegt.

Zu diesem Zweck kann die dentale Restauration 100 gezielt mit Kräften belastet werden, die während des Einsatzes der dentalen Restauration 100 auftreten können. Diese greifen an den schwächsten Punkten der dentalen Restauration 100 an. Ist die dentale Restauration 100 beispielsweise eine Brücke, so wird diese mit der maximal auftretenden Kraft in der Mitte der Brücke beaufschlagt. Die FEM-Software berechnet dann, wie sich die Spannungen innerhalb der Brücke verteilen. Auf diese Weise lassen sich räumliche Bereiche 101-1 bestimmen, in denen die Spannungen besonders hoch sind.

Es können aber auch anhand einer realen Bisssituation real auftretende Kräfte mittels einer Druckmatte bestimmt werden. Die Druckmatte misst auftretende Kräfte beim Zusammenbeißen der Zähne. Die ermittelten Kräfte können von der FEM-Software dann für die dentale Restauration 100 verwendet werden, um die interne Verteilung der Spannung innerhalb der dentalen Restauration 100 unter realen Bedingungen zu berechnen.

Die FEM-Software ist zudem in der Lage, Empfehlungen für eine Materialanpassung zu geben, da diese die mechanischen Eigenschaften der unterschiedlichen verfügbaren Herstellungsmaterialien 107-1 und 107-2 kennt. Dadurch dass die unter- und/oder überbestimmten räumlichen Bereiche 101-1 und 101-2 durch die FEM-Analyse in der dentalen Restauration 100 bekannt sind, können entsprechende Korrekturmaßnahmen getroffen werden. Dadurch kann die Eintrittswahrscheinlichkeit eines Bauteilversagens minimiert und eine Belastungsgarantie für den Kunden abgegeben werden. Weiter ermöglicht die Software eine Plausibilitätsprüfung der verwendeten Herstellungsmaterialien 107-1 und 107-2.

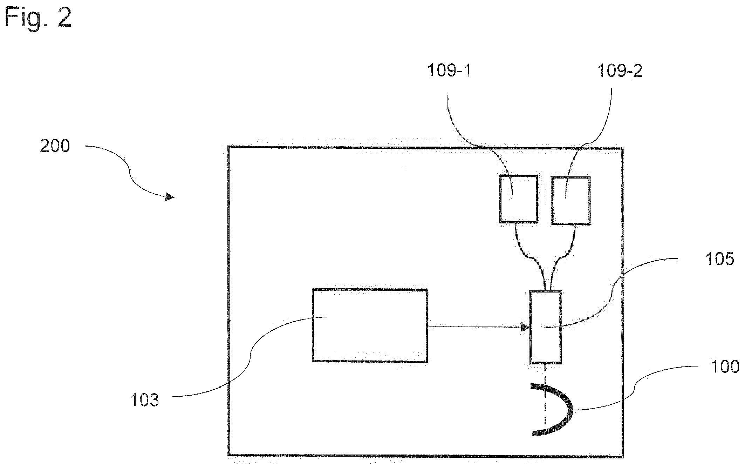

Fig. 2 zeigt eine schematische Ansicht einer Herstellungsvorrichtung 200 zum Herstellen der dentalen Restauration 100. Die Herstellungsvorrichtung 200 umfasst eine Bestimmungsvorrichtung 103 zum Bestimmen eines ersten räumlichen Bereiches 101-1 der dentalen Restauration 100, der einer höheren Belastung ausgesetzt ist als ein zweiter räumlicher Bereich 101-2 der dentalen Restauration 100.

Die Bestimmungsvorrichtung 103 umfasst beispielsweise einen Prozessor und einen Speicher, auf dem eine FEM-Software ausgeführt wird. Die Bestimmungsvorrichtung 103 ist beispielsweise durch einen Computer gebildet.

Die FEM-Software, die auf der Bestimmungsvorrichtung 103 läuft, berechnet anhand eines digitalen Modells der dentalen Restauration 100 diejenigen räumlichen Bereiche, in den höhere mechanische Belastungen auftreten als in anderen räumlichen Bereichen. Auf diese Weise kann das digitale Modell der dentalen Restauration 100 in unterschiedliche räumliche Bereiche 101-1 oder 101-2 aufgeteilt werden. Dabei können auch drei oder mehr räumliche Bereiche mit unterschiedlichen mechanischen Belastungen innerhalb der dentalen Restauration 100 festgelegt werden. Für jeden dieser räumlichen Bereiche kann ein eigenes Herstellungsmaterial verwendet werden.

Anschließend sendet Bestimmungsvorrichtung 103 Steuerdaten an ein Herstellungsgerät 105, mit dem die dentale Restauration hergestellt wird. Die Bestimmungsvorrichtung 103 kann je nach den berechneten Belastungen unterschiedlichen räumlichen Bereichen 101-1 und 101-2 automatisch die jeweils zu verwendeten Herstellungsmaterialien 107-1 und 107-2 zuordnen. Dies kann durchgeführt werden, da die Bestimmungsvorrichtung 103 die Eigenschaften der verfügbaren Herstellungsmaterialien kennt.

Das Herstellungsgerät 105 verwendet in dem ersten räumlichen Bereich 101-1 ein anderes Herstellungsmaterial 107-1 als in dem zweiten räumlicher Bereich 101-2. Diese Herstellungsmaterial 107-1 kann eine höhere Festigkeit als das andere Herstellungsmaterial 107-2 aufweisen.

Das Herstellungsgerät 103 verwendet beispielsweise einen additiven Fertigungsprozess, bei dem ein Rohmaterial in Form von Tropfen selektiv schichtweise abgelegt wird, wie beispielsweise ein Poly-Jet Modelling und das Multi-Jet Modelling. Das Herstellungsgerät 105 umfasst beispielsweise einen ersten Behälter 109-1 für das erste Herstellungsmaterial 101-1 und einen zweiten Behälter 109-2 für das zweite Herstellungsmaterial 107-2. Die Steuersignale von der Bestimmungsvorrichtung veranlassen das Herstellungsgerät 105 beim Drucken des jeweiligen räumlichen Bereichs 101-1 und 101-2, automatisch des zugeordnete Herstellungsmaterial 107-1 oder 107-2 zu verwenden.

Durch die Herstellungsvorrichtung 200 kann die Isotropie der Lastverteilung innerhalb der dentalen Restauration 100 verringert werden. Es kann ein anderes, wie beispielsweise festeres Herstellungsmaterial 107-1 für einen stärker beanspruchten oder belasteten räumlichen Bereich 101-1 verwendet werden, wie beispielsweise Zirkonoxid mit 3 Gewichtsprozent Yttriumoxid (3YTZP) statt Zirkonoxid mit 5 Gewichtsprozent Yttriumoxid (5YTZP).

Würde für beide räumlichen Bereiche 101-1 und 101-2 das gleiche Herstellungsmaterial 107 verwendet, würde dies eine Aufdickung der dentalen Restauration 100 erfordern, die aus ästhetischen und haptischen Gründen oft nicht erwünscht ist. Die Herstellungsvorrichtung 200 ermöglicht eine Verringerung der Wandstärke bei unterbelasteten Subvolumen für eine bessere Ästhetik.

Diese selektive Anpassung der Materialzuordnung kann im Rahmen eines additiven Multimaterial-Fertigungsprozesses durchgeführt werden, bei dem ein selektiver Materialauftrag frei im dreidimensionalen Raum erfolgt, wie beispielsweise in einen Freistrahl-Materialauftrag beim Poly- oder Multi-Jet-Modelling.

Bei einem 3D-Inkjet-Printing können beispielsweise Herstellungsmaterialien 107-1 und 107-2 mit einer Viskosität größer als 15 mPas, vorzugsweise größer als 150 mPas und hoch vorzugsweise größer als 200 mPas bei einer geeigneten Verarbeitungstemperatur verjettet werden. Durch den selektiven Materialauftrag des Material-Jettings können kleinste räumliche Bereiche 101-1 bis zu einer Größe von einem einzigen Voxel mit unterschiedlichen Herstellungsmaterialien 107-1 und 107-2 aufgebaut werden.

Wird durch einen Benutzer entweder das Design der dentalen Restauration 100 geändert oder andere Herstellungsmaterialien 107-1 und 107-2 mit anderen mechanischen Eigenschaften zugeordnet, kann diese Veränderung für den Benutzer sichtbar gemacht werden. Sind das Design und die Herstellungsmaterialien 107-1 und 107-2 in Ordnung, kann der Benutzer den Druckauftrag für die dentale Restauration 100 starten. Diese wird dann automatisch mit den jeweiligen Herstellungsmaterialien 107-1 und 107-2 in den jeweiligen räumlichen Bereichen 101-1 und 101-2 aufgebaut.

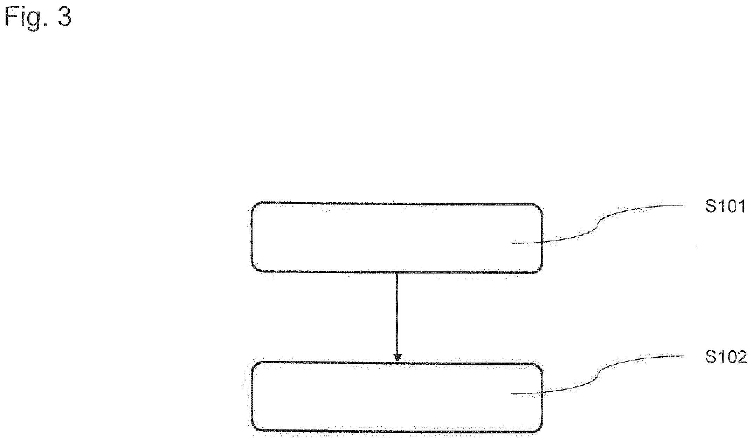

Fig. 3 zeigt ein Blockdiagramm eines Verfahrens zum Herstellen der dentalen Restauration 100. In Schritt S101 wird ein erster räumlicher Bereich 101-1 der dentalen Restauration 100 bestimmt, der einer höheren Belastung ausgesetzt ist als ein zweiter räumlicher Bereich 101-2 der dentalen Restauration 100. In Schritt S102 wird die dentale Restauration 100 in dem ersten räumlichen Bereich 101-1 mit einem anderen Herstellungsmaterial 107-1 als in dem zweiten räumlichen Bereich 101-2 hergestellt. Das Verfahren kann auf alle räumlichen Bereiche 101-1 und 101-2 der dentalen Restauration 100 angewendet werden.

Bei der Verarbeitung von Zirkonoxidschlickern können lokal die Sinter-Eigenschaften in einem räumlichen Bereich dadurch verändert werden, dass die Materialschicht im Grünlingsstadium selektiv unterschiedlich dotiert wird. Dies führt im Sinterprozess bei Verwendung von verschiedenen Herstellungsmaterialen 107-1 und 107-2 in der finalen dentalen Restauration 100 zu unterschiedlichen Festigkeitsbereichen. Die selektive Dotierung erfolgt beispielsweise über das Verjetten einer Infiltrationsflüssigkeit in eine zuvor aufgetragene und getrocknete Materialschicht. Neben den Sintereigenschaften können auch die mechanischen Eigenschaften, die optischen Eigenschaften und die Alterungsbeständigkeit mittels der Dotierung eingestellt werden.

Durch das Verfahren können räumliche Bereiche 101 in der dentalen Restauration 100 identifiziert werden, die entweder überbestimmt sind oder verstärkt werden sollten.

Dementsprechend können selektiv und lokal andere Herstellungsmaterialien 107-1 und 107-2 mit anderen mechanischen Eigenschaften zugewiesen werden. Dadurch können Kronen oder Brücken aus finalem Herstellungsmaterial 107-1 und 107-2 (Composite oder Keramik), den individuellen Lastfällen angepasst werden.

Der Umfang der Indikationen kann ausgeweitet werden und die auftretende Belastung quantifiziert und sichtbar gemacht werden. Der Zahntechniker kann bei einer Modellierung der dentalen Restauration unterstützt werden, indem räumliche Bereiche 101 angezeigt werden, an denen die Wandstärken verringert werden können oder erhöht werden sollten. Durch Zuordnung verschiedener Herstellungsmaterialien 107-1 und 107-2 mit unterschiedlichen mechanischen Eigenschaften zu den jeweiligen räumlichen Bereichen 101 können Lastspitzen ohne zusätzliche Erhöhung der Wandstärke abgefangen werden.

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Alle Verfahrensschritte können durch Vorrichtungen implementiert werden, die zum Ausführen des jeweiligen Verfahrensschrittes geeignet sind. Alle Funktionen, die von gegenständlichen Merkmalen ausgeführt werden, können ein Verfahrensschritt eines Verfahrens sein.

Der Schutzbereich der vorliegenden Erfindung ist durch die Ansprüche gegeben und wird durch die in der Beschreibung erläuterten oder den Figuren gezeigten Merkmale nicht beschränkt.

### BEZUGSZEICHENLISTE

- 100: Dentale Restauration
- 101: Räumlicher Bereich
- 103: Bestimmungsvorrichtung
- 105: Herstellungsvorrichtung
- 107: Herstellungsmaterial
- 109: Behälter

## Patentansprüche

1. Nicht therapeutisches Verfahren zum Herstellen einer dentalen Restauration (100), mit den Schritten:
- Bestimmen (S101) eines ersten räumlichen Bereiches (101-1) der dentalen Restauration (100), der einer höheren mechanischen Belastung ausgesetzt ist als ein zweiter räumlicher Bereich (101-2) der dentalen Restauration (100); und
- Herstellen (S102) der dentalen Restauration (100) in dem ersten räumlichen Bereich (101-1) mit einem anderen Herstellungsmaterial (107-1) als in dem zweiten räumlicher Bereich (101-2); **dadurch gekennzeichnet, dass** das Herstellungsmaterial (107-1) in dem ersten räumlichen Bereich (101-1) anders als in dem zweiten räumlichen Bereich (101-2) dotiert wird.

2. Verfahren nach Anspruch 1, wobei der erste räumliche Bereich (101-1) dadurch bestimmt wird, dass in diesem räumlichen Bereich (101-1) die interne Spannung über einem vorgegebenen Wert liegt.

3. Verfahren nach Anspruch 2, wobei die Belastung mittels einer Finite-Elemente-Methode berechnet wird.

4. Verfahren nach Anspruch 3, wobei vorgegebene Kräfte auf die dentale Restauration (100) für die Finite-Elemente-Methode angewendet werden.

5. Verfahren nach einem der vorangehenden Ansprüche, wobei das Herstellungsmaterial (107-1, 107-2) für den ersten und/oder zweiten räumlichen Bereich (101-1, 101-2) auf Basis einer berechneten Belastung ausgewählt wird.

6. Verfahren nach einem der vorangehenden Ansprüche, wobei in dem ersten räumlichen Bereich (101-1) ein Herstellungsmaterial (107-1) mit einer höheren Festigkeit als in dem zweiten räumlichen Bereich verwendet wird.

7. Verfahren nach einem der vorangehenden Ansprüche, wobei das Herstellen der dentalen Restauration (100) mittels eines dreidimensionalen Druckverfahrens durchgeführt wird.

8. Verfahren nach Anspruch 7, wobei das dreidimensionale Druckverfahren einen Freistrahl-Materialauftrag verwendet.

9. Herstellungsvorrichtung (200) zum Herstellen einer dentalen Restauration (100), mit:
- einer Bestimmungsvorrichtung (103) zum Bestimmen eines ersten räumlichen Bereiches (101-1) der dentalen Restauration (100), der einer höheren mechanischen Belastung ausgesetzt ist als ein zweiter räumlicher Bereich (101-2) der dentalen Restauration (100); und
- einem Herstellungsgerät (105) zum Herstellen der dentalen Restauration (100) in dem ersten räumlichen Bereich (101-1) mit einem anderen Herstellungsmaterial (107-1) als in dem zweiten räumlicher Bereich (101-2), **dadurch gekennzeichnet, dass** das Herstellungsgerät (105) ausgebildet ist, das Herstellungsmaterial (107-1) in dem ersten räumlichen Bereich (101-1) anders als in dem zweiten räumlichen Bereich (101-2) zu dotieren.

10. Herstellungsvorrichtung (200) nach Anspruch 9, wobei die Bestimmungsvorrichtung (103) ausgebildet ist, den ersten räumlichen Bereich (101-1) dadurch zu bestimmen, dass in diesem räumlichen Bereich (101-1) die interne Spannung über einem vorgegebenen Wert liegt.

11. Herstellungsvorrichtung (200) nach Anspruch 10, wobei die Bestimmungsvorrichtung (103) ausgebildet ist, die Belastung mittels einer Finite-Elemente-Methode zu berechnen.

12. Herstellungsvorrichtung (200) nach einem der Ansprüche 9 bis 11, wobei das Herstellungsgerät (105) ausgebildet ist, das Herstellungsmaterial (107-1, 107-2) für den ersten und/oder zweiten räumlichen Bereich (101-1, 101-2) auf Basis einer berechneten Belastung auszuwählen.

13. Herstellungsvorrichtung (200) nach einem der Ansprüche 9 bis 12, wobei das Herstellungsgerät (105) einen 3D-Drucker umfasst.

## Claims

1. A non-therapeutic method of producing a dental restoration (100), comprising the steps of:
- determining (S101) a first spatial region (101-1) of the dental restoration (100) that is subject to a higher mechanical load than a second spatial region (101-2) of the dental restoration (100); and
- producing (S102) the dental restoration (100) in the first spatial region (101-1) with a different production material (107-1) than in the second spatial region (101-2); **characterized in that** the production material (107-1) in the first spatial region (101-1) is doped differently than in the second spatial region (101-2).

2. The method according to claim 1, wherein the first spatial region (101-1) is determined by the internal stress in this spatial region (101-1) being above a predetermined value.

3. The method according to claim 2, wherein the load is calculated using a finite element method.

4. The method according to claim 3, wherein predetermined forces are applied to the dental restoration (100) for the finite element method.

5. The method according to any one of the preceding claims, wherein the production material (107-1, 107-2) for the first and/or second spatial region (101-1, 101-2) is selected based on a calculated load.

6. The method according to any one of the preceding claims, wherein in the first spatial region (101-1) a production material (107-1) having a higher strength than in the second spatial region is used.

7. The method according to any one of the preceding claims, wherein the production of the dental restoration (100) is performed by means of a three-dimensional printing process.

8. The method according to claim 7, wherein the three-dimensional printing process uses free-jet material deposition.

9. A production device (200) for producing a dental restoration (100), comprising:
- a determination device (103) for determining a first spatial region (101-1) of the dental restoration (100) which is subject to a higher mechanical load than a second spatial region (101-2) of the dental restoration (100); and
- a production apparatus (105) for producing the dental restoration (100) in the first spatial region (107-1) with a different production material (107-1) than in the second spatial region (101-2), **characterized in that** the production apparatus (105) is configured to dope the production material (107-1) in the first spatial region (101-1) differently than in the second spatial region (101-2).

10. The production device (200) according to claim 9, wherein the determination device (103) is configured to determine the first spatial region (101-1) by the internal stress being above a predetermined value in this spatial region (101-1).

11. The production device (200) according to claim 10, wherein the determination device (103) is configured to calculate the load using a finite element method.

12. The production device (200) according to any one of claims 9 to 11, wherein the production apparatus (105) is configured to select the production material (107-1, 107-2) for the first and/or second spatial region (101-1, 101-2) based on a calculated load.

13. The production device (200) according to any one of claims 9 to 12, wherein the production apparatus (105) comprises a 3D printer.

## Revendications

1. Procédé non thérapeutique de réalisation d'une restauration dentaire (100), comprenant les étapes consistant à :
- déterminer (S101) une première région spatiale (101-1) de la restauration dentaire (100) qui est soumise à une contrainte mécanique plus élevée qu'une deuxième région spatiale (101-2) de la restauration dentaire (100) ; et
- fabriquer (S102) la restauration dentaire (100) dans la première région spatiale (101-1) avec un matériau de fabrication différent (107-1) que dans la deuxième région spatiale (101-2) ; **caractérisé en ce que** le matériau de fabrication (107-1) est dopé différemment dans la première région spatiale (101-1) que dans la deuxième région spatiale (101-2).

2. Procédé selon la revendication 1, où la première région spatiale (101-1) est déterminée par le fait que dans cette région spatiale (101-1), la contrainte interne est supérieure à une valeur prédéterminée.

3. Procédé selon la revendication 2, où la contrainte est calculée au moyen d'une méthode d'éléments finis.

4. Procédé selon la revendication 3, où des forces prédéterminées sont appliquées à la restauration dentaire (100) pour la méthode d'éléments finis.

5. Procédé selon l'une des revendications précédentes, où le matériau de fabrication (107-1, 107-2) est sélectionné pour la première et/ou la deuxième région spatiale (101-1, 101-2) sur la base d'une contrainte calculée.

6. Procédé selon l'une des revendications précédentes, où dans la première région spatiale (101-1) un matériau de fabrication (107-1) ayant une résistance plus élevée que dans la deuxième région spatiale est utilisé.

7. Procédé selon l'une des revendications précédentes, où la préparation de la restauration dentaire (100) est effectuée au moyen d'un procédé d'impression tridimensionnelle.

8. Procédé selon la revendication 7, où le procédé d'impression tridimensionnelle utilise une application de matériau à jet libre.

9. Dispositif de fabrication (200) pour la réalisation d'une restauration dentaire (100), comprenant :
- un dispositif de détermination (103) pour déterminer une première région spatiale (101-1) de la restauration dentaire (100) qui est exposée à une contrainte mécanique plus élevée qu'une deuxième région spatiale (101-2) de la restauration dentaire (100) ; et
- un appareil de fabrication (105) pour la préparation d'une restauration dentaire (100) dans la première région spatiale (101-1) avec un matériau de fabrication (107-1) différent de celui utilisé dans la deuxième région spatiale (101-2),
**caractérisé en ce que** l'appareil de fabrication (105) est conçu pour doper le matériau de fabrication (107-1) dans la première région spatiale (101-1) différemment que dans la deuxième région spatiale (101-2).

10. Dispositif de fabrication (200) selon la revendication 9, où le dispositif de détermination (103) est conçu pour déterminer la première région spatiale (101-1) par le fait que dans cette région spatiale (101-1), la contrainte interne est supérieure à une valeur prédéterminée.

11. Dispositif de fabrication (200) selon la revendication 10, où le dispositif de détermination (103) est conçu pour calculer la contrainte au moyen d'une méthode par éléments finis.

12. Dispositif de fabrication (200) selon l'une des revendications 9 à 11, où l'appareil de fabrication (105) est conçu pour sélectionner le matériau de fabrication (107-1, 107-2) pour la première et/ou la deuxième région spatiale (101-1, 101-2) sur la base d'une contrainte calculée.

13. Dispositif de fabrication (200) selon l'une des revendications 9 à 12, où l'appareil de fabrication (105) comprend une imprimante 3D.
